# EUROPEAN PATENT SPECIFICATION

(11) **EP 2 692 282 B1**
(45) Date of publication and mention of the grant of the patent: **19.11.2014**
(21) Application number: 13177504.1
(22) Date of filing: 22.07.2013
(51) Int. Cl.: A61B 1/31, A61B 1/32

(54) **Anoscope**
Anoskop
Anuscope

(30) Priority: 02.08.2012 IT MI20121377
(43) Date of publication of application: 05.02.2014
(73) Proprietor: Angiologica B.M. S.R.L., 27028 S. Martino Siccomario (IT)
(72) Inventor: Manca, Antonio, 27100 Pavia (IT)
(74) Representative: Cernuzzi, Daniele

(56) References cited:
- EP-A1- 1 929 959
- WO-A1-2004/021874
- US-A- 2 769 441
- US-A- 2 922 415
- US-A- 6 142 931
- US-A1- 2006 009 797

## Description

The present invention concerns an anoscope, in particular a disposable sterile rotating anoscope, which can be used, among other things, in proctology as a diagnostic and/or surgical instrument, and is particularly suitable for the surgical treatment of haemorrhoids.
An anoscope is a device which is used for performing examinations and operations in various medical areas, such as general medicine, gastroenterology, proctology, endoscopy and general surgery.
In general, an anoscope has a hollow body, provided with a handgrip, purposely shaped and generically tapered towards one end to allow introduction into the anal region of the patient and provided with an internal longitudinal cavity which allows inspection and access to the area by the doctor.

A sector of particular use of anoscopes is for the diagnosis and treatment of haemorrhoids.

A particularly simple, quick and effective technique for the surgical treatment of haemorrhoids consists in transanal haemorrhoidal deartelization; essentially, this technique consists in repositioning of the haemorrhoidal tissue in its anatomical position by means of anorectal sutures and simultaneous ligature of the branches of the superior haemorrhoidal artery.

For the performance of this technique (among others), an anoscope of the type described for example in the patent application WO2004/021874 is commonly used.

This type of anoscope comprises a fixed part, provided with a handgrip and which remains in contact with the anoderm, and a rotating operating part, which is inserted inside the fixed part and projects frontally therefrom and comes into contact with the mucous membrane of the anal canal; the rotating part is hollow and provided with a window through which the sutures are performed and can be manipulated by means of an external ring so as to reach the various positions in which to perform the sutures.

The anoscopes of this type, but also others with analogous structure and without the rotating part, are also provided with a dilator/introducer, which can be inserted inside the hollow body of the anoscope during positioning and manipulation of the instrument in the anal region of the patient. The dilator is shaped so as to dilate the intestinal lumen and facilitate entry of the anoscope. Once the anoscope has been positioned, the dilator is removed, thus making the intestinal lumen accessible to the operator; the dilator is re-introduced when the anoscope has to be manipulated (for example, by rotating the rotatable part), repositioned or extracted, to distend the rectal mucous membrane and therefore facilitate movement of the instrument.

The dilator is introduced via an aperture at the rear end of the anoscope and normally abuts against an internal shoulder of the anoscope, so as to project from the front end of the anoscope for a preset fixed length.

This solution has the main drawback that the anoscope with the dilator inserted has a fixed length and therefore cannot be adapted to different operating requirements, for example according to the patient or the type of operation to be performed.

More generally, the known anoscopes are subject to improvement, especially in terms of effectiveness, versatility and simplicity of production and use.

It is an object of the present invention to provide an anoscope that overcomes the drawbacks of the known art; in particular, an object of the invention is to provide a particularly versatile and effective anoscope, which is simple to produce and use.

The present invention therefore relates to an anoscope as claimed in claim 1.

In a preferred embodiment, the stop element is ring-shaped and is for example made of elastomeric material, in particular silicone, and is set around the body of the dilator and is mechanically coupled to the body with radial interference, so as to assume firmly each operating position and be moved along the body subject to a preset force.

According to one aspect of the invention, furthermore, the base body is formed of a fixed part and a movable part, which rotates with respect to the fixed part around the axis and projects axially from the fixed part at both axially opposite ends of the base body, respectively with an operating portion provided with a lateral window and with a ring that rests axially against a rear collar of the fixed part; the ring is provided with a reference element which is aligned with the window parallel to the axis, so as to signal to the operator the angular position of the window.

For example, the reference element is a visual mark, in particular made with non-toxic indelible ink, and/or a notch or projection.

The anoscope of the invention is particularly versatile, as it can be simply and quickly adapted to different operating needs; the anoscope of the invention is furthermore simple to produce and use and fully effective, in particular in the performance of surgical operations for the treatment of haemorrhoids and specifically the technique of transanal haemorrhoidal deartelization.

Further characteristics and advantages of the present invention will appear clear from the following description of a non-limiting embodiment example thereof, with reference to the figures of the accompanying drawings, in which:
- figure 1 is a lateral schematic view of an anoscope according to the invention;
- figure 2 is an overhead view of a first component of the anoscope of figure 1, and specifically of a base body of the anoscope;
- figure 3 is a rear view of the component of figure 2;
- figure 4 is a lateral view of a further component of the anoscope of figure 1, and specifically of a dilator.

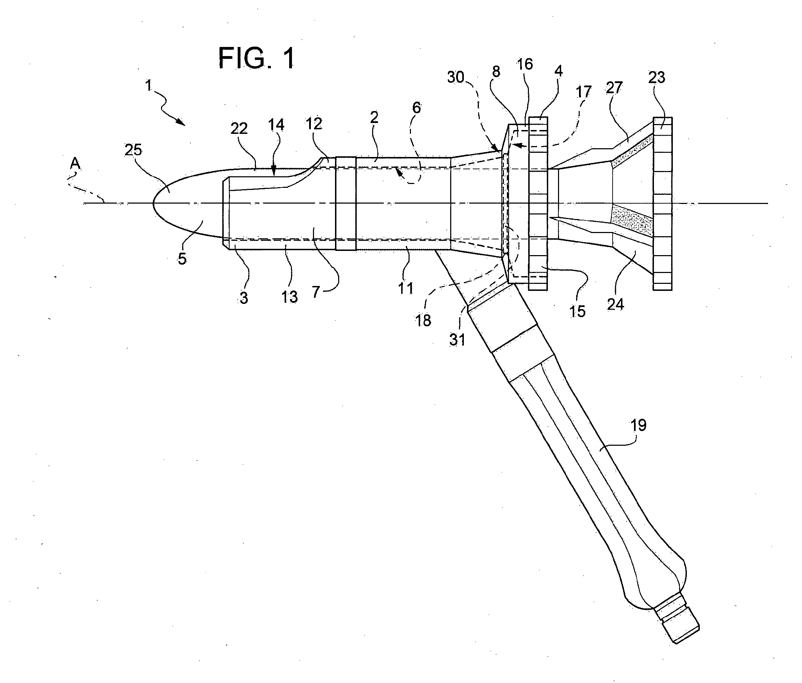

In figure 1 the number 1 indicates as a whole an anoscope, in particular a disposable sterile anoscope.

The anoscope 1 comprises a hollow base body 2, which extends along an axis A between a front end 3 and a rear end 4, and a dilator 5, which can be inserted inside the base body 2.

The end 3 is the end of the anoscope 1 which, in use, is introduced into the rectal canal, while the end 4 is the end facing in use towards the operator.

The base body 2 is hollow inside, having a through internal longitudinal cavity 6 between the ends 3, 4; the cavity 6 extends along the axis A and is open at the opposite ends 3, 4 of the base body 2.

The base body 2 has a shape generically tapered towards the front end 3, specifically comprising a front end portion 7 shaped so as to penetrate into the rectal canal via the anal orifice, and a manipulation portion 8, positioned at the rear end 4 and having a transverse section greater than the front end portion 7.

In the non-limiting example illustrated, the base body 2 is formed of a fixed part 11 and a movable part 12, rotating with respect to the fixed part 11 about the axis A.

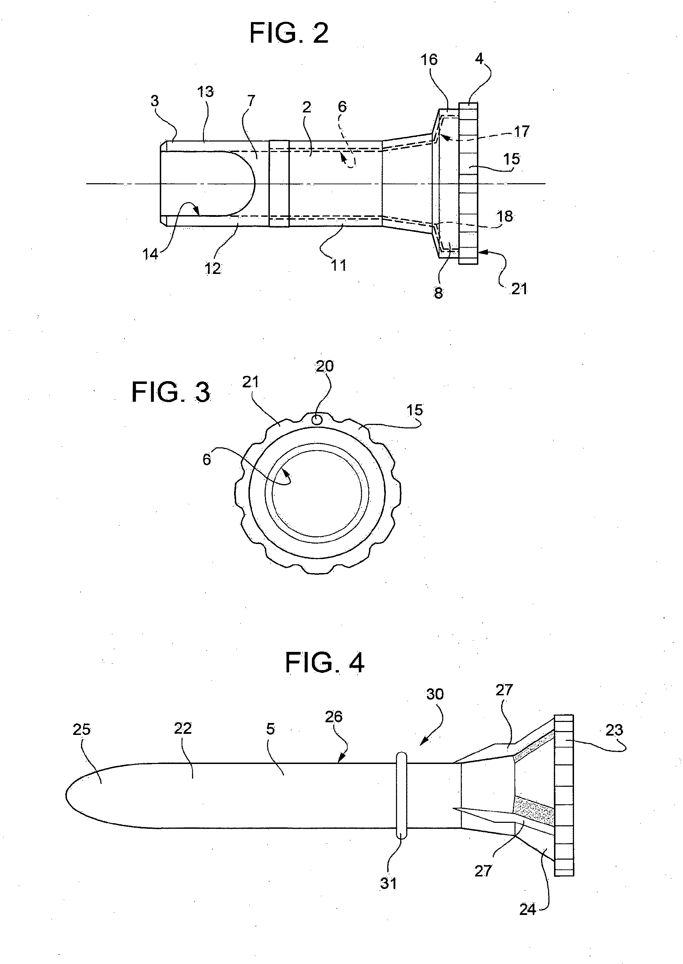

With reference also to the figures 2 and 3, the movable part 12 is inserted in the fixed part 11 and is axially blocked inside the fixed part 11 and coupled in a rotating manner with the fixed part 11 so as to rotate about the axis A with respect to the fixed part 11; the movable part 12 projects axially outside the fixed part 11 at both the axially opposite ends 3, 4 of the base body 2, respectively with an operating portion 13 provided with a lateral window 14 (formed in a lateral wall of the portion 13) and with a positioning ring 15 which axially abuts against a rear collar 16 of the fixed part 11.

The fixed part 11 and the movable part 12 are both internally hollow so as to define together the cavity 6.

The cavity 6 includes a portion 17 flared towards the end 4.

The cavity 6 is internally provided with an axial shoulder 18, facing towards the rear end 4 and defined for example by a variation of the cross section of the cavity 6, for example of the flared portion 17.

Preferably, the movable part 12 is made of a transparent rigid polymer material, for example transparent polycarbonate; the fixed part 1, on the other hand, is made of an opaque (non-transparent) rigid polymer material, for example polyamide (nylon).

The base body 2 is provided with a handgrip 19, for example made of polyamide, which projects obliquely, for example, from the fixed part 12.

The positioning ring 15 of the movable part 12 is provided with a reference element 20 (for example, a visual mark, in particular made with non-toxic indelible ink, and/or a notch or projection), arranged for example on a rear ring-shaped surface 21 of the positioning ring 15 and aligned axially (parallel to the axis A) with the window 14, so as to signal to the operator the angular position of the window 14.

With reference also to figure 4, the dilator 5, advantageously made of a transparent rigid polymer material, for example transparent polycarbonate, has a shape elongated longitudinally along the axis A and such as to be insertable in a removable manner in the cavity 6 and to protrude axially from the ends 3, 4 of the base body 2.

In particular, the dilator 5 comprises an oblong body 22, sliding inside the cavity 6, and a radially external flange 23, positioned at a first axial end 24 of the dilator 5 corresponding to the rear end 4 of the base body 2.

The body 21 has a second axial end 25, opposite the end 24 and intended to project, in use, axially from the front end 3 of the base body 2; the end 25 is tapered and/or provided with a rounded tip.

The body 21 has a substantially cylindrical lateral surface 26 which extends on at least one portion of the body 21.

Optionally, the flange 23 is connected to the body 22 by means of fins or ribs 27 having a profile corresponding to the flared portion 17 of the cavity 6.

The anoscope 1 is provided with a position adjustment device 30, which allows adjustment of the position of the dilator 5 inside the base body 2, namely in the cavity 6, and therefore adjustment of the length of the portion of the dilator 5 which projects from the end 3 of the base body 2.

The device 30 comprises a stop element 31, which projects radially from the lateral surface 26 and abuts against the shoulder 18; the stop element 31 is movable on the body 22 along the axis A to stop selectively in any one of a plurality of operating positions on the body 21.

In the non-limiting example illustrated, the stop element 31 is ring-shaped and set around the body 22; for example, the stop element 31 is a ring made of elastomeric material, in particular silicone; the stop element 31 is mechanically coupled with the body 22 with radial interference, so as to assume firmly each operating position and be moved along the body 22 subject to a preset force; in other words, the stop element 31 is coupled by interference with the body 22 and slides along the body 22, on the lateral surface 26, only if subject to a preset axial force.

It is understood that the stop element 31 can have another shape, and be coupled to the body 22 in another way.

In use, when the operator has to position the anoscope 1, the operator adjusts the position of the stop element 31 on the body 22 of the dilator 5, then inserts the dilator 5 into the cavity 6 of the base body 2; the dilator 5 slides in the cavity 6 until the stop element 31 abuts against the shoulder 18; at this point, the operator introduces the anoscope 1, provided with the dilator 5, through the anal region of the patient.

Adjustment of the dilator 5 allows use of the anoscope 1 to be adapted to specific needs, for example according to the patient or the type of operation to be performed.

The operator then rotates, if necessary, the movable part 12, by means of the positioning ring 15, bringing the window 14 to the desired position, which will be signalled to the operator by the position of the reference element 20.

Once the anoscope 1 is correctly positioned, the dilator 5 is extracted.

Lastly it is understood that the anoscope described and illustrated here can be subject to further modifications and variations which do not depart from the scope of the attached claims.

## Claims

1. An anoscope (1), comprising a hollow base body (2), that extends along an axis (A) between a front end (3) and a rear end (4) and has a through internal longitudinal cavity (6) between the ends (3, 4); a dilator (5), having a shape elongated longitudinally along the axis (A) and such as to be removably insertable in the cavity (6) and to project axially from the ends (3, 4); and a position adjusting device (30) for adjusting the position of the dilator (5) inside the cavity (6) and varying the length of the portion of the dilator (5) projecting from the front end (3) of the base body (2); wherein the device (30) comprises a stop element (31), that projects radially from the dilator (5) and axially abuts against an axial shoulder (18) formed in the cavity (6), **characterized in that** the stop element (31) is movable on a body (22) of the dilator (5) along the axis (A) for selectively stopping in any one of a plurality of operating positions on the body (22).

2. An anoscope according to claim 1, wherein the stop element (31) is ring-shaped and is set around the body (22) of the dilator (5) and is mechanically coupled to the body (22) with radial interference, so as to assume firmly each operating position and be moved along the body (22) subject to a preset force.

3. An anoscope according to claim 1 or 2, wherein the stop element (31) is a ring made of an elastomeric material, in particular of silicone.

4. An anoscope according to one of the preceding claims, wherein the base body (2) is formed of a fixed part (11) and a movable part (12), that rotates with respect to the fixed part (11) about the axis (A) and projects axially from the fixed part (11) at both axially opposite ends (3, 4) of the base body (2), respectively with an operating portion (13), provided with a lateral window (14), and with a positioning ring (15) that rests axially against a rear collar (16) of the fixed part (11); the positioning ring (15) being provided with a reference element (20) that is aligned with the window (14) parallel to the axis (A), so as to signal to the user the angular position of the window (14).

5. An anoscope according to claim 4, wherein the reference element (20) includes a visual mark, in particular made with a non-toxic indelible ink, and/or a notch or a projection.

6. An anoscope according to claim 4 or 5, wherein the movable part (12) is made of a transparent rigid polymer material, for example transparent polycarbonate; and the fixed part (1) is made of an opaque rigid polymer material, for example polyamide.

7. An anoscope according to one of the preceding claims, wherein the dilator (5) is made of a transparent rigid polymer material, for example transparent polycarbonate.

## Patentansprüche

1. Anoskop (1), das umfasst: einen hohlen Grundkörper (2), der sich längs einer Achse (A) zwischen einem vorderen Ende (3) und einem hinteren Ende (4) erstreckt und einen durchgängigen inneren Hohlraum (6) in Längsrichtung zwischen den Enden (3, 4) aufweist; einen Dilatator (5), der eine Form hat, die sich in Längsrichtung längs der Achse (A) ausdehnt und in den Hohlraum (6) entnehmbar eingesetzt werden kann und von den Enden (3, 4) axial vorsteht; und eine Positionseinstellvorrichtung (30) zum Einstellen der Position des Dilatators (5) im Inneren des Hohlraums (6) und zum Ändern der Länge des Abschnitts des Dilatators (5), der von dem vorderen Ende (3) des Grundkörpers (2) vorsteht,; wobei die Vorrichtung (30) ein Anschlagelement (31) umfasst, das von dem Dilatator (5) radial vorsteht und axial an einer axialen Schulter (18) anliegt, die in dem Hohlraum (6) ausgebildet ist, **dadurch gekennzeichnet, dass** das Anschlagelement (31) auf einem Körper (22) des Dilatators (5) längs der Achse (A) bewegt werden kann, um es wahlweise in einer von mehreren Arbeitspositionen auf dem Körper (22) anzuhalten.

2. Anoskop nach Anspruch 1, wobei das Anschlagelement (31) ringförmig ist und um den Körper (22) des Dilatators (5) gelegt ist und mit dem Körper (22) in radialem Eingriff mechanisch gekoppelt ist, um jede Arbeitsposition festsitzend einnehmen zu können und längs des Körpers (22) bewegt werden zu können, wenn es einer zuvor eingestellten Kraft ausgesetzt ist.

3. Anoskop nach Anspruch 1 oder 2, wobei das Anschlagelement (31) ein Ring ist, der aus einem Elastomermaterial, insbesondere aus Silikon hergestellt ist.

4. Anoskop nach einem der vorhergehenden Ansprüche, wobei der Grundkörper (2) aus einem feststehenden Bauteil (11) und einem beweglichen Bauteil (12), das sich in Bezug auf das feststehende Bauteil (11) um die Achse (A) dreht und von dem feststehenden Bauteil (11) an beiden axial entgegengesetzten Enden (3, 4) des Grundkörpers (2) axial vorsteht, jeweils mit einem Arbeitsabschnitt (13), der mit einem seitlichen Fenster (14) versehen ist, und mit einem Positionierungsring (15), der axial auf einem hinteren Kragen (16) des feststehenden Bauteils (11) ruht, gebildet ist; wobei der Positionierungsring (15) mit einem Bezugselement (20) versehen ist, das parallel zu der Achse (A) auf das Fenster (14) ausgerichtet ist, um dem Anwender die Winkelposition des Fensters (14) zu signalisieren.

5. Anoskop nach Anspruch 4, wobei das Bezugselement (20) eine sichtbare Markierung, die insbesondere mit einer ungiftigen wischfesten Tinte hergestellt ist, und/oder eine Kerbe oder einen Vorsprung aufweist.

6. Anoskop nach Anspruch 4 oder 5, wobei das bewegliche Bauteil (12) aus einem lichtdurchlässigen starren Polymermaterial, beispielsweise aus lichtdurchlässigem Polycarbonat, hergestellt ist; und das feststehende Bauteil (11) aus einem lichtundurchlässigen starren Polymermaterial, beispielsweise aus Polyamid, hergestellt ist.

7. Anoskop nach einem der vorhergehenden Ansprüche, wobei der Dilatator (5) aus einem lichtdurchlässigen starren Polymermaterial, beispielsweise aus lichtdurchlässigem Polycarbonat, hergestellt ist.

## Revendications

1. Anuscope (1), comprenant un corps de base creux (2), qui s'étend le long d'un axe (A) entre une extrémité avant (3) et une extrémité arrière (4) et présente une cavité longitudinale interne traversante (6) entre les extrémités (3, 4) ; un dilatateur (5), ayant une forme allongée longitudinalement le long de l'axe (A) et de telle sorte à pouvoir être inséré de manière amovible dans la cavité (6) et à faire saillie axialement à partir des extrémités (3, 4) ; et un dispositif de réglage de position (30) pour régler la position du dilatateur (5) à l'intérieur de la cavité (6) et modifier la longueur de la partie du dilatateur (5) faisant saillie à partir de l'extrémité avant (3) du corps de base (2) ; dans lequel le dispositif (30) comprend un élément de butée (31), qui fait saillie radialement à partir du dilatateur (5) et vient en butée axialement contre un épaulement axial (18) formé dans la cavité (6), **caractérisé en ce que** l'élément de butée (31) est mobile sur un corps (22) du dilatateur (5) le long de l'axe (A) pour s'arrêter sélectivement dans l'une quelconque d'une pluralité de positions d'utilisation sur le corps (22).

2. Anuscope selon la revendication 1, dans lequel l'élément de butée (31) est de forme annulaire et est fixé autour du corps (22) du dilatateur (5), et est couplé mécaniquement au corps (22) avec une interférence radiale, de sorte à occuper fermement chaque position d'utilisation et à être déplacé le long du corps (22) soumis à une force préétablie.

3. Anuscope selon la revendication 1 ou 2, dans lequel l'élément de butée (31) est un anneau composé d'un matériau élastomère, en particulier de silicone.

4. Anuscope selon l'une des revendications précédentes, dans lequel le corps de base (2) est formé d'une partie fixe (11) et d'une partie mobile (12), qui tourne par rapport à la partie fixe (11) autour de l'axe (A) et fait saillie axialement à partir de la partie fixe (11) au niveau des deux extrémités axialement opposées (3, 4) du corps de base (2), respectivement, avec une partie d'utilisation (13) pourvue d'une fenêtre latérale (14), et avec un anneau de positionnement (15) qui repose axialement contre un collier arrière (16) de la partie fixe (11) ; l'anneau de positionnement (15) étant pourvu d'un élément de référence (20) qui est aligné avec la fenêtre (14) parallèle à l'axe (A), de sorte à indiquer à l'utilisateur la position angulaire de la fenêtre (14).

5. Anuscope selon la revendication 4, dans lequel l'élément de référence (20) comporte un repère visuel, en particulier réalisé avec une encre indélébile non toxique, et/ou une encoche ou une saillie.

6. Anuscope selon la revendication 4 ou 5, dans lequel la partie mobile (12) est composée d'un matériau polymère rigide transparent, par exemple, de polycarbonate transparent ; et la partie fixe (1) est composée d'un matériau polymère rigide opaque, par exemple, de polyamide.

7. Anuscope selon l'une des revendications précédentes, dans lequel le dilatateur (5) est composé d'un matériau polymère rigide transparent, par exemple, de polycarbonate transparent.
